# EUROPEAN PATENT APPLICATION

(11) **EP 4 364 671 A1**
(43) Date of publication of application: **08.05.2024**
(21) Application number: 22315260.4
(22) Date of filing: 03.11.2022
(51) Int. Cl.: A61B 8/00

(54) **SYSTEMS FOR ULTRASOUND IMAGING, METHODS FOR ULTRASOUND IMAGING , AND COMPUTER PROGRAM PRODUCTS**

(71) Applicant: Caranx Medical SAS, 75013 Paris (FR)
(72) Inventor: Jonas Victor Harmen SMITS, 3360 Bierbeek (BE); Cerruti, Giulio, 06700 Saint-Laurent-Du-Var (FR); Mira, Anna, 06000 Nice (FR)
(74) Representative: Hepp Wenger Ryffel AG

(57) **Abstract**

A system (1) for ultrasound imaging is disclosed. The system comprises an ultrasound probe (2) with a probe surface (3) adapted to be directed towards a patient's skin (S); a moveable mount (4) for holding, moving, and positioning the ultrasound probe (2); a mount control (5) for automated movement and positioning of the mount (4); a coupling unit (6) configured to be arranged between the probe surface (3) and the patient's skin (S), thereby defining a contact medium area (7) for receiving a contact medium (10); a contact medium control (8) for the control of at least one characteristic of a contact medium (10) located in the contact medium area (7); and an acoustic coupling assessment tool (9) for assessing an acoustic coupling between the probe surface (3) and the patient's skin and for generating a coupling parameter. The system (1) is adapted to operate the contact medium control (8) based on the coupling parameter. Method for automated ultrasound imaging and related computer program products are also disclosed.

## Description

The present invention pertains to systems for ultrasound imaging, methods for ultrasound imaging, and computer program products.

WO 2019/147940 A1 discloses methods and apparatuses for ultrasound coupling. Certain aspects relate to coupling bodies for acoustically coupling an ultrasound device to a subject. A coupling body may include a first surface configured to couple to an ultrasound device, a second surface configured to contact the subject, a reservoir internal to the coupling body, and a plurality of openings extending between the reservoir and one or both of the first surface and the second surface. The reservoir may contain ultrasound gel. A coupling body may include an adhesive coupled to a subpart of the surface of the coupling body. A coupling body may include a first surface configured to contact the ultrasound device and a second surface including first adhesive configured to adhere to the subject. The first surface may also include second adhesive configured to adhere to an ultrasound patch device. Certain aspects also relate to packaging coupling bodies.

US 2006/0030778 A1 discloses methods and devices for reducing medical probe contamination by providing rigid probe holders.

US 2005/0096547 A1 discloses an accommodative standoff holder which can be used for both diagnostic and therapeutic ultrasound is disclosed. The described standoff holder has the ability to be mounted on and utilized with transducers of different sizes and shapes. The standoff holder includes a gel insert which is removable and self-adjusting assuring contact between a human body, an animal, or other object and the acoustic window of a transducer. Further, a gel insert and method of making gel inserts from a gel pad is described.

WO 2008/151277 A1 discloses methods and apparatus for dispensing fluid in an apparatus for applying acoustic energy to the skin. Acoustic waveguides are disclosed which compensate for distortions that otherwise occur when a focused acoustic beam crosses a boundary, such as the transition from a treatment device to a target region of skin. The invention is especially useful with devices that focus ultrasound energy by condensing a propagating wavefront. The invention compensates for the mismatch in acoustic properties of the device's waveguide and the biological tissue that typically cause portions of the collapsing wavefront to lag behind other portions and, thereby, limit the focusing capabilities of acoustic treatment devices.

It is an object of the present invention to provide improved systems, methods, and computer programs for ultrasound imaging. In particular, they should obtain and maintain a desirable and predetermined level of acoustic coupling between an ultrasound probe and a patient's skin. Additionally, in certain embodiments of the invention, acoustic coupling quality during scanning operations should be optimized or maximally maintained. More specifically, the effects of influential factors such as scanning motion, contact force variation, contact medium degradation, surface type variation, etc.

In one aspect, the invention pertains to a system for ultrasound imaging which comprises an ultrasound probe with a probe surface adapted to be directed towards a patient's skin; a moveable mount for holding, moving and positioning the ultrasound probe; a mount control for automated movement and positioning of the mount; a coupling unit configured to be arranged between the probe surface and the patient's skin, thereby defining a contact medium area for receiving a contact medium; a contact medium control for the control of at least one characteristic of a contact medium located in the contact medium area; an acoustic coupling assessment tool for assessing an acoustic coupling between the probe surface and the patient's skin and for generating a coupling parameter. The system is adapted to operate the contact medium control based on the coupling parameter.

Within the present specification, the term "contact medium" is used to describe any type of fluid with acoustic coupling impedance. Moreover, the term "characteristic of a contact medium" refers to any characteristic relevant for the acoustic coupling condition. It can be a property of the contact medium as such (for example presence, amount, or thickness), but also an external parameter such as pressure on the ultrasound probe or an under-pressure in a chamber. In addition, the parameter may be a resistance which measures connectivity variability between contact and non-contact states.

With the system according to the invention, a predetermined level of acoustic coupling between an ultrasound probe and a patient's skin can be securely obtained and maintained even when influential factors such as scanning motion, contact force variation, contact medium degradation, surface type variation, etc. occur.

In some embodiments, the acoustic coupling assessment tool is adapted to automatically analyze the image quality of an ultrasonic image taken by the ultrasound probe, whereby the coupling parameter is generated. This automatic analysis is reliable and precise. It may be performed by employing image processing techniques, in particular confidence maps. For example, horizontal non-uniformity may be detected to determine bubbles in the contact medium. Alternatively or in addition, vertical non-uniformity on the edges of the image may be used for detecting probe pressure not orthogonal to the skin surface.

In some embodiments, the acoustic coupling assessment tool is adapted to analyze the contact medium area, whereby the coupling parameter is generated.

The acoustic coupling assessment tool may be adapted to determine a volume of the contact medium area not filled with contact medium. In particular, it may determine (i) inclusions of gas within the contact medium and/or (ii) areas free from contact medium neighboring the probe surface or the patient's skin.

In some embodiments, the acoustic coupling assessment tool is adapted to determine the volume of the contact medium area which is not filled with contact medium. This may be performed, for example, by measuring electrical conductivity within the contact medium area or by image analysis. Image analysis (e.g. by using a confidence map) can be used to determine if shadows are present in the image, e.g. if air is present in the coupling medium or part of the probe scanning area is not in contact with the skin.

Alternatively, the compressibility of the material (the contact medium with potential gas enclosures) could be measured, noting that the compressibility increases with increasing gas enclosures. Based on different compressibility of liquid or gaseous material, the pressure/volume relationship changes in function of the ratio between gas (in particular air) and liquid. This allows to detect the presence of (compressible) air volumes in an overall sealed volume.

The characteristic of the contact medium controlled by the contact medium control may be the pressure applied by the ultrasound probe onto the contact medium. In these embodiments, the contact medium control may be adapted to control the pressure applied by the ultrasound probe on the patient's skin.

In some embodiments, the coupling unit further comprises an automated supply for automated supply of the contact medium. The characteristic of the contact medium controlled by the contact medium control may be the amount of contact medium provided in the contact medium area. The contact medium control may be adapted to control the amount of contact medium supplied by the automated supply of the contact medium.

In some embodiments, the coupling unit further comprises a suction unit for creating an under-pressure within the contact medium area. The characteristic of the contact medium controlled by the contact medium control may be the volume of the contact medium area not filled with contact medium. The contact medium control may be adapted to control operation of the suction unit.

In some embodiments, the coupling unit further comprises a sealing member surrounding the contact medium area for sealingly contacting the patient's skin.

In some embodiments, the automated supply for automated supply of the contact medium may be an active dispensing unit, in particular with a pump or a plunger. The release (flow and location) can be operated in open-loop, for example time-based, or in closed-loop, i.e., based on feedback of one or multiple sensors.

The pump could be implemented using any standard solution for volume displacement of viscous fluid, including a syringe driver, a peristaltic pump, an Archimedes screw, piston pump, etc. The pump draws contact medium from a reservoir. The reservoir may be equipped with an inlet valve to allow air entry to offset the volume flow and resulting pressure drop. In the case of a syringe driver, such a valve can be omitted (reservoir volume change in function of flow).

Alternatively, the automated supply for automated supply of the contact medium may be a passive dispensing unit, in particular a dispensing unit with at least one dispensing opening openable upon contact between the probe surface and the patient's skin. The dispensing unit may comprise movable elements such as rollers.

The system may further comprise a containment feature which is mechanically decoupled from the ultrasound probe, for example by means of an elastic element. In the absence of external forces acting on the ultrasound probe, the containment feature may seal a reservoir in which the contact medium is held. As force is exerted on the ultrasound probe or on the containment feature, relative motion between the containment feature and the ultrasound probe may occur, thereby opening the reservoir and releasing the contact medium. Relative motion can be linear or angular.

In some embodiments, the system further comprises at least one sensor for sensing an additional parameter. The additional parameter may be a motion and/or position of the ultrasound probe which includes the speed and position with respect to patient's skin. In particular, the distance to skin or a contact with skin may be detected. Direction and speed, collected by means of a IMU (inertial measurement unit) fixed to the probe or by means of an external positioning device.

Alternatively, the additional parameter may be a pressure acting on the ultrasound probe. In these embodiments, the system may be further adapted to operate the contact medium control based on the additional parameter.

In some embodiments, the system further comprises a reservoir for a contact medium, wherein the reservoir is preferably unitary with the movable mount for the ultrasound probe.

In some embodiments, the system further comprises a holder for a medical tool, wherein the holder is preferably unitary with the movable mount for the ultrasound probe.

The invention also encompasses a method for automated ultrasound imaging, preferably with a system as described above. The method comprises the steps of
- moving and positioning an ultrasound probe with a probe surface directed towards a patient's skin by controlling an automated movement and positioning of a movable mount carrying the ultrasound probe,
- placing a coupling unit between the probe surface and the patient's skin, thereby defining a contact medium area,
- automatically supplying a contact medium within the contact medium area,
- assessing an acoustic coupling between the probe surface and the patient's skin with an acoustic coupling assessment tool, thereby generating a coupling parameter,
- controlling at least one characteristic of the contact medium located in the contact medium area based on the coupling parameter.

Placing the coupling unit is preferably performed simultaneously with moving and positioning the ultrasound probe. Alternatively, placing the coupling unit could be performed individually.

Furthermore, the invention pertains to a computer program product which directly loadable into the internal memory of a digital computer or stored.on a computer usable medium. The computer program product comprises software code portions for performing the steps listed below when said product is run on a computer which is in operative connection with (i) a mount control for automated movement and positioning of a moveable mount for holding, moving and positioning an ultrasound probe having a probe surface and (ii) a contact medium control for the control of at least one characteristic of a contact medium. The steps mentioned above are
- controlling the mount control such as to move and position the ultrasound probe with the probe surface directed towards a patient's skin,
- automatically providing a contact medium within a contact medium area between the probe surface and the patient's skin,
- controlling at least one characteristic of the contact medium located in the contact medium area based on a coupling parameter.

The invention and its advantages will be further explained on the basis of the following examples and drawings, in which
- Figure 1: shows a schematic drawing of a first embodiment;
- Figure 2: shows a schematic drawing of a detail of the first embodiment;
- Figure 3: shows a schematic drawing of a detail of a second embodiment;
- Figure 4: shows a schematic drawing of a detail of a third embodiment;
- Figure 5: shows a schematic drawing of a detail of a fourth embodiment;
- Figure 6: shows a schematic drawing of a detail of a fifth embodiment;
- Figure 7: shows a schematic drawing of a detail of a sixth embodiment;
- Figure 8: shows a schematic drawing of a detail of a seventh embodiment.

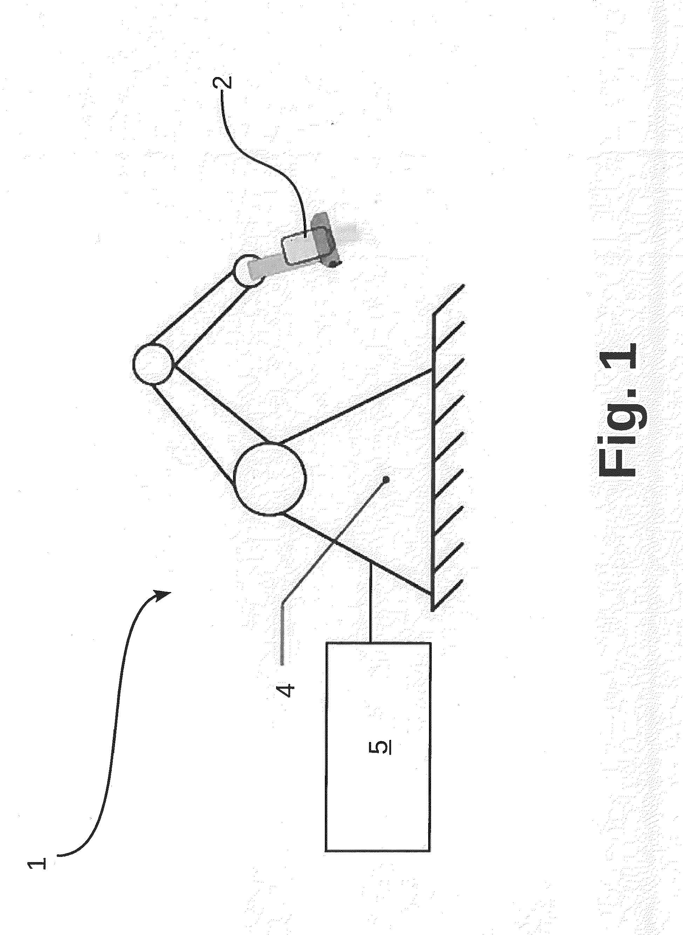

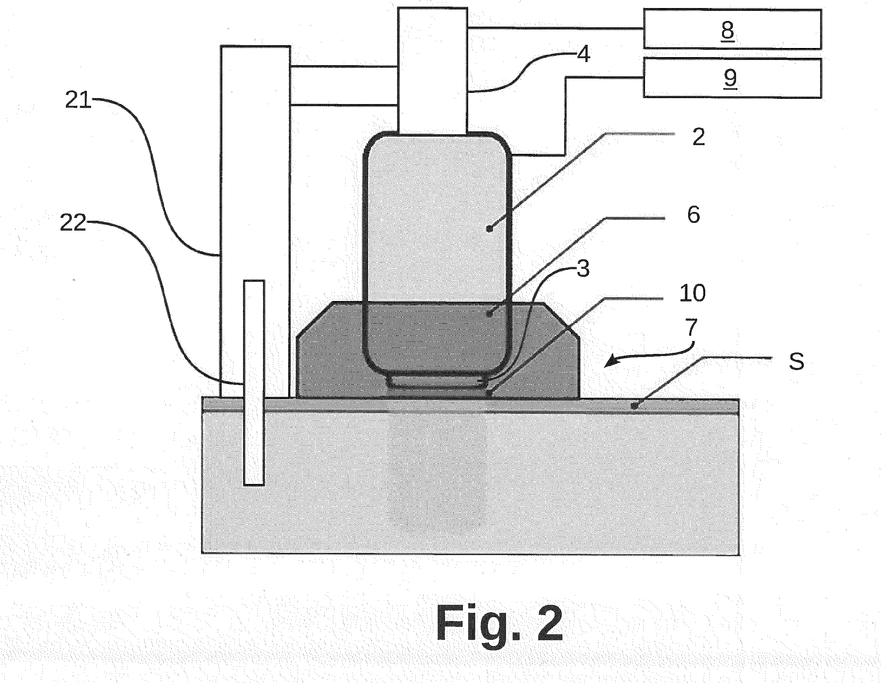

The system 1 depicted in Figure 1 comprises an ultrasound probe 2 and a movable mount 4 for holding, moving, and positioning the ultrasound probe 2, a mount control 5 for automated movement and positioning of the mount 4, a coupling unit configured to be arranged between a probe surface 3 and a patient's skin (see Figure 2).

The detailed view in Figure 2 shows a probe surface 3 of the ultrasound probe 2 which is directed towards a patient's skin S. A coupling unit 6 is arranged between the probe surface 3 and the patient's skin S and thereby defines a contact medium area 7 for receiving a contact medium 10, for example a contact gel. The system 1 further comprises an acoustic coupling assessment tool 9 for assessing an acoustic coupling between the probe surface 3 and the skin S and for generating a coupling parameter. A contact medium control 8 control the characteristic of the contact medium 10 that is located in the contact medium area 7. A holder 21 which is unitary with the movable mount 4 holds a medical tool 22 which is inserted into the skin S.

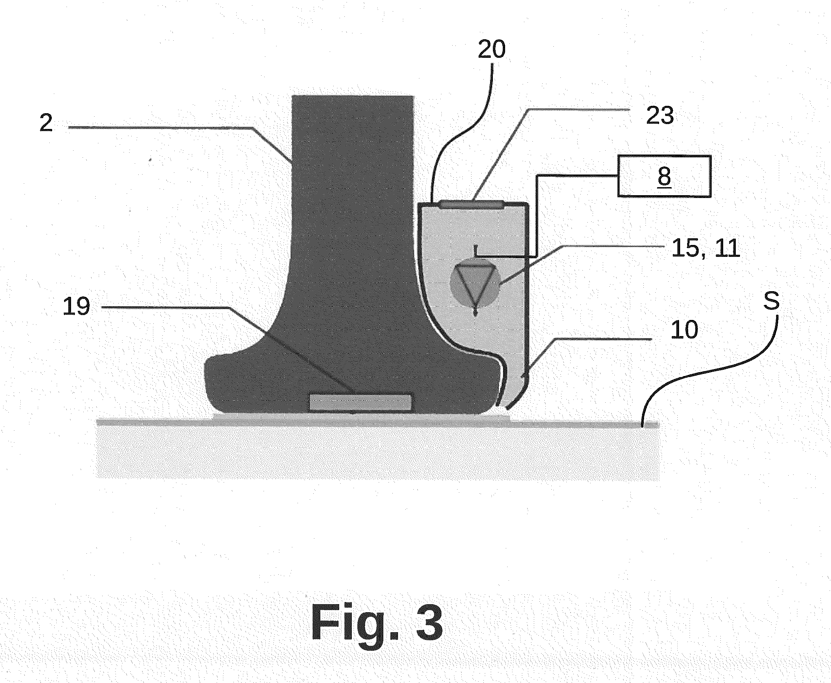

The embodiment shown in Figure 3 contains a pump 15 for actively dispensing the contact medium 10 at specific target sites and with controlled flow, thus providing an automated supply 11 for the contact medium 10. The pump 15 is controlled by a contact medium control 8, which allows to control the contact medium 10 in the contact medium area 7. Instead of a pump 15, a plunger could also be employed. The system 1 further contains an inlet valve 23 for allowing air entry. In addition, the system 1 comprises a sensor 19 for sensing an additional parameter. The additional parameter may be a motion and/or position of the ultrasound probe 2, in particular the speed and/or the position of the ultrasound probe 2 with respect to the skin S.

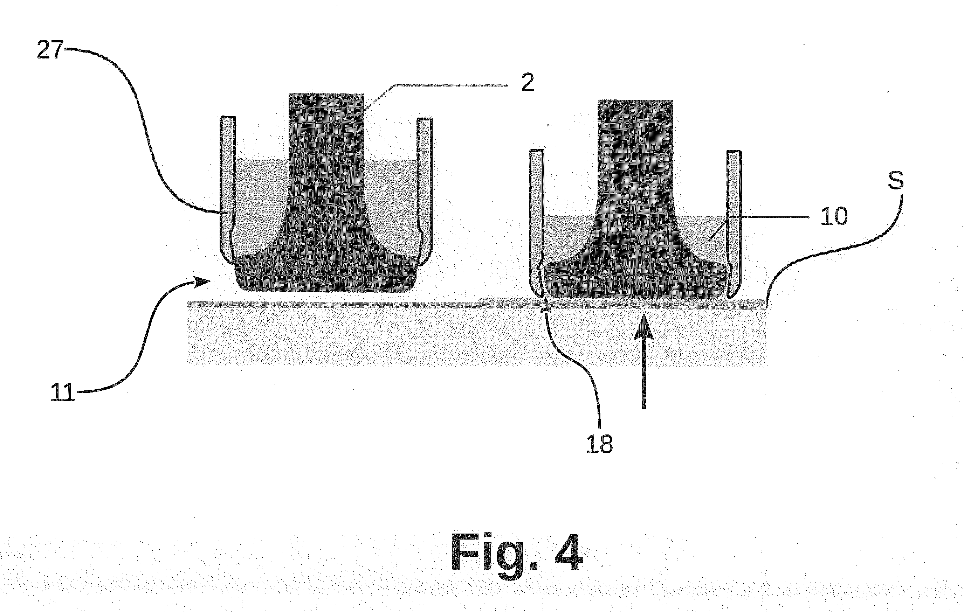

Figure 4 shows a further embodiment in which the automated supply is a passive dispensing unit 17. The system 1 comprises a containment feature 27 which is mechanically decoupled from the ultrasound probe 2 by means of an elastic element. In the absence of external forces acting on the ultrasound probe 2, the containment feature 27 seals the reservoir 20. As a force is exerted on the ultrasound probe 2 or on the containment feature 27, relative motion between the containment feature 27 and the ultrasound probe 2 occurs, opening the reservoir 20 and releasing the contact medium 10 through a dispensing opening 18. Relative motion can be linear, as shown in Figure 4, or angular.

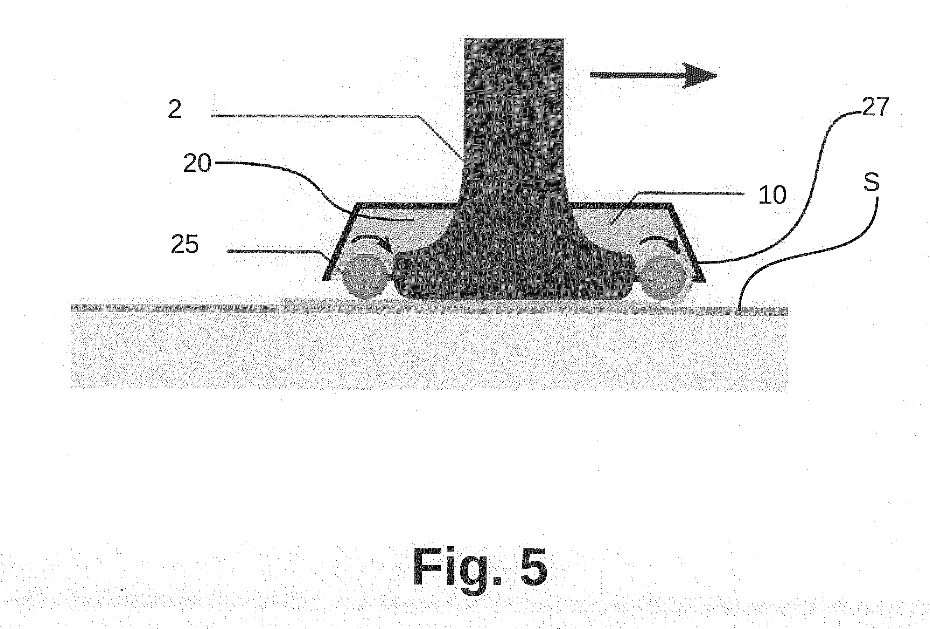

In the embodiment shown in Figure 5, a containment feature 27 is fixed to the ultrasound probe 2. A contact medium 10 is dispensed by moving elements fixed to the containment feature 27. These elements could be one or a set of mono or omnidirectional elements, for example rollers 25 as shown in Figure 5 or, alternatively, spheres. The rollers 25 may be actively driven. The contact medium 10 is retrieved from the reservoir 20 by means of friction. Additionally, the rollers 25 could have an uneven surface shape to enhance spreading of the contact medium 10 and ensuring contact with the skin S at the same time.

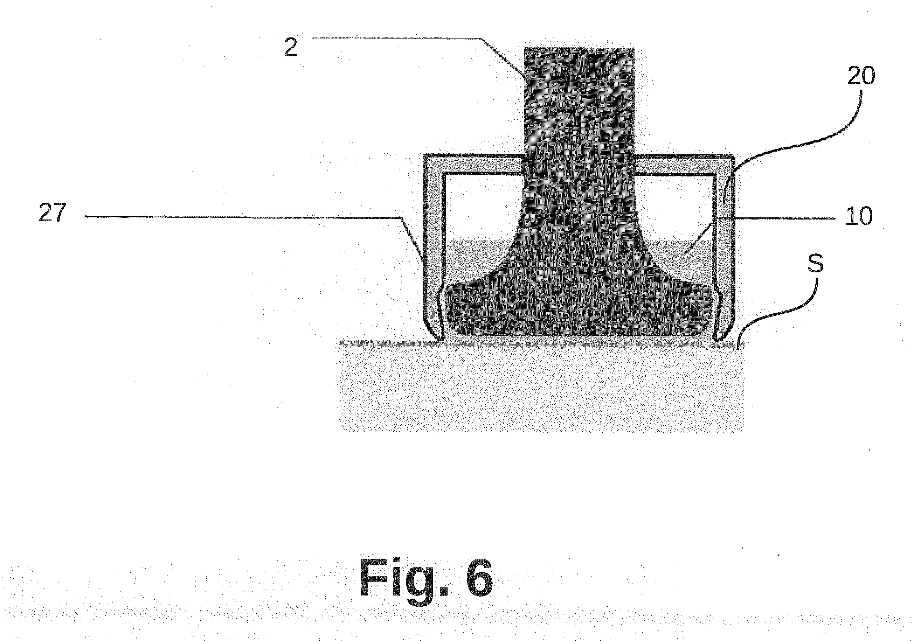

In the embodiment shown in Figure 6, a containment feature 27 is used to passively control dispensing of the contact medium 10 around the interface between contact surface 3 and skin S by leaving a small mechanical opening at the sides of the ultrasound probe 2. Additionally, the layer of the contact medium 10 between the skin S and the ultrasound probe 2 outside of the containment zone could be controlled by means of spreading elements, like brushes or squeegees, at the edge of the containment feature 27.

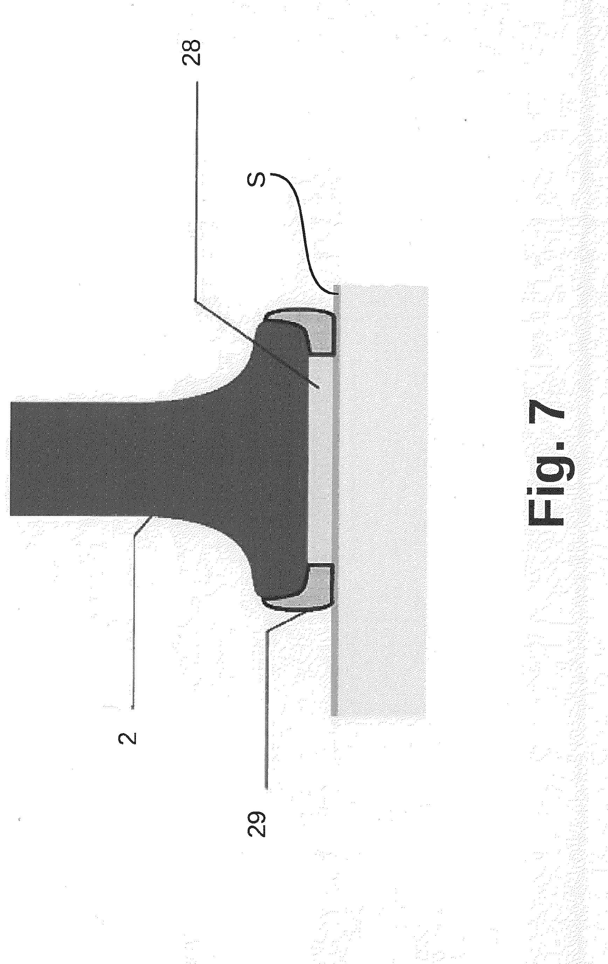

In the embodiment shown in Figure 7, a pre-loaded coupling pad 28 (or, alternative, at least one capsule(s)) poses the initial coupling interface between ultrasound probe 2 and skin S. The coupling pad 28 can either be a solid interface which remains so, or a solid interface which may be dissolvable when exposed to saline or water (e.g., dehydrated water-soluble coupling medium).

An additional irrigation feature could be present (not shown), allowing for local application of water or saline solution at the scanning region and probe-tissue interface. It is readily understood that such an irrigation feature can be easily included using solutions well known in the field, such as syringe drivers, pumps, drip tubes with an elevated bag, etc.

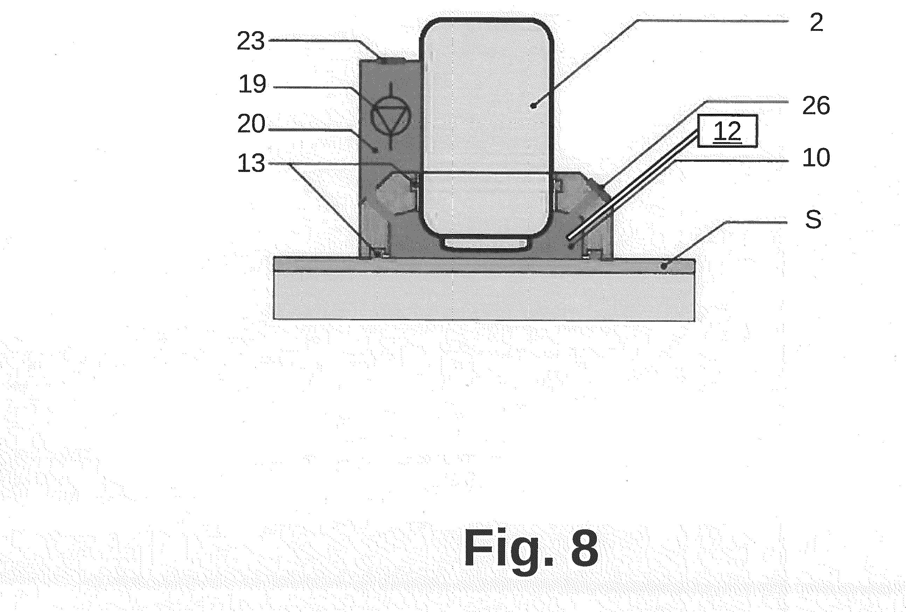

The embodiment shown in Figure 8 contains a pump 15 which draws contact medium 10 from a reservoir 20. The reservoir 20 is equipped with an inlet valve 23 to allow air entry to offset the volume flow and resulting pressure drop. In addition to the inlet valve 23, an outlet valve 26 is also present. Furthermore, the system comprises sealing members 13.

Furthermore, through pressure regulation, transducer-tissue contact can be .improved by setting the reservoir 20 to a minor under-pressure by a suction unit 12. This creates a local suction effect, ensuring air is removed from the transducer-tissue interface. Furthermore, a contact force is generated between the ultrasound probe 2 and the skin S, benefitting the contact condition during scanning motion as well as other simultaneous tissue interactions in near vicinity of the scanning field (e.g., needle puncture, instrument insertions, etc.).

## Claims

1. A system (1) for ultrasound imaging, the system comprising
- an ultrasound probe (2) with a probe surface (3) adapted to be directed towards a patient's skin (S),
- a moveable mount (4) for holding, moving, and positioning the ultrasound probe (2),
- a mount control (5) for automated movement and positioning of the mount (4),
- a coupling unit (6) configured to be arranged between the probe surface (3) and the patient's skin (S), thereby defining a contact medium area (7) for receiving a contact medium (10),
- a contact medium control (8) for the control of at least one characteristic of a contact medium (10) located in the contact medium area (7),
- an acoustic coupling assessment tool (9) for assessing an acoustic coupling between the probe surface (3) and the patient's skin and for generating a coupling parameter,
wherein the system (1) is adapted to operate the contact medium control (8) based on the coupling parameter.

2. The system (1) according to claim 1,
wherein the acoustic coupling assessment tool (9) is adapted to automatically analyze the image quality of an ultrasonic image taken by the ultrasound probe (2), thereby generating the coupling parameter.

3. The system (1) according to claim 1,
wherein the acoustic coupling assessment tool (9) is adapted to analyze the contact medium area (7), thereby generating the coupling parameter.

4. The system (1) according to claim 3,
wherein the acoustic coupling assessment tool (9) is adapted to determine a volume of the contact medium area (7) not filled with contact medium (10),
in particular to determine (i) inclusions of gas within the contact medium (10) and/or (ii) areas free from contact medium (10) neighboring the probe surface (3) or the patient's skin (S).

5. The system (1) according to claim 4,
wherein the acoustic coupling assessment tool (9) is adapted to determine the volume of the contact medium area (7) not filled with contact medium (10) based on at least one of image analysis, conductivity of the contact medium area, and compressibility of the contact medium area.

6. The system (1) according to one of the claims 1 to 5,
wherein the characteristic of the contact medium (10) controlled by the contact medium control (8) is the pressure applied by the ultrasound probe (2) onto the contact medium (10) and wherein the contact medium control (8) is adapted to control the pressure applied by the ultrasound probe (2) on the patient's skin (S).

7. The system (1) according to one of the claims 1 to 6, the coupling unit (6) further comprising an automated supply (11) for automated supply of the contact medium (10), wherein the characteristic of the contact medium (10) controlled by the contact medium control (8) is the amount of contact medium (10) provided in the contact medium area (7) and
wherein contact medium control (10) is adapted to control the amount of contact medium (10) supplied by the automated supply (11) of the contact medium (10).

8. The system (1) according to one of the claims 4 to 7,
the coupling unit (6) further comprising a suction unit (12) for creating an underpressure within the contact medium area,
wherein the characteristic of the contact medium (10) controlled by the contact medium control (8) is the volume of the contact medium area (7) not filled with contact medium (10) and
wherein the contact medium control (8)is adapted to control operation of the suction unit (12) .

9. The system (1) according to claim 8, the coupling unit (6) further comprising a sealing member (13) surrounding the contactmedium area (7) for sealingly contacting the patient's skin (S) .

10. The system (1) according to one of the claims 7 to 9, wherein the automated supply (11) for automated supply of the contact medium (10) is selected from the group of
- an active dispensing unit (14), in particular with a pump (15) or a plunger,
- a passive dispensing unit (17), in particular a dispensing unit (17) with at least one dispensing opening (18) openable upon contact between the probe surface (3) and the patient's skin (S).

11. The system (1) according to one of the claims 1 to 10, further comprising at least one sensor (19) for sensing an additional parameter selected from
- a motion and/or position of the ultrasound probe (2) with respect to the patient, in particular distance and/or contact to the patient's skin,
- a pressure acting on the ultrasound probe (2), wherein the system (1) is further adapted to operate the contact medium control (8) based on the additional parameter.

12. The system (1) according to one of the claims 1 to 11, further comprising a reservoir (20) for a contact medium (10), the reservoir (20) preferably being unitary with the movable mount (4) for the ultrasound probe (2).

13. The system (1) according to one of the claims 1 to 12, further comprising a holder (21) for a medical tool (22), the holder (21) preferably being unitary with the movable mount (4) for the ultrasound probe (2).

14. A method for automated ultrasound imaging, preferably with a system (1) according to one of the claims 1 to 13, the method comprising the steps of
- moving and positioning an ultrasound probe (2) with a probe surface (3) directed towards a patient's skin (S) by controlling an automated movement and positioning of a movable mount (4) carrying the ultrasound probe (2),
- placing a coupling unit (6) between the probe surface (3) and the patient's skin (S), thereby defining a contact medium area (7),
- automatically supplying a contact medium (10) within the contact medium area (7),
- assessing an acoustic coupling between the probe surface (3) and the patient's skin (S) with an acoustic coupling assessment tool (9), thereby generating a coupling parameter,
- controlling at least one characteristic of the contact medium (10) located in the contact medium area (7) based on the coupling parameter.

15. A computer program product directly loadable into the internal memory of a digital computer or stored on a computer usable medium, comprising software code portions for performing the following steps when said product is run on a computer which is in operative connection with (i) a mount control (5) for automated movement and positioning of a moveable mount (4) for holding, moving and positioning an ultrasound probe (2) having a probe surface (3) and (ii) a contact medium control (8) for the control of at least one characteristic of a contact medium:
- controlling .the mount control (5) such as to move and position the ultrasound probe (2) with the probe surface (3) directed towards a patient's skin (S),
- automatically providing a contact medium (10) within a contact medium area (7) between the probe surface (3) and the patient's skin (S),
- controlling at least one characteristic of the contact medium (10) located in the contact medium area (7) based on a coupling parameter.
